# EUROPEAN PATENT APPLICATION

(11) **EP 2 108 956 A1**
(43) Date of publication of application: **14.10.2009**
(21) Application number: 08154164.1
(22) Date of filing: 07.04.2008
(51) Int. Cl.: G01N 33/574

(54) **Improved methods and kits for detecting tumor-specific fusion proteins**

(71) Applicant: Erasmus University Medical Center Rotterdam, 3015 GD Rotterdam (NL)
(72) Inventor: The designation of the inventor has not yet been filed
(74) Representative: Habets, Winand

(57) **Abstract**

The invention relates to the field of cancer diagnosis and the application of diagnostic techniques in pathology and hematology. Specifically, the invention relates to improved flow cytometric techniques, and kits related thereto, for the detection of chromosomal aberrations and the detection of tumor specific gene products exclusively expressed by tumor cells.

## Description

The invention relates to the field of cancer diagnosis and the application of diagnostic techniques in pathology and hematology. Specifically, the invention relates to flow cytometric techniques, and kits related thereto, for the detection of chromosomal aberrations and the detection of tumor specific gene products exclusively expressed by tumor cells containing said chromosomal aberrations, wherein a sample is contacted with at least two different probes directed against the fusion protein, each probe being reactive with a distinct site on the fusion protein.

Detection methods of this type are known in the art. For example, US 6,686,165 describes a rapid multiplexed, bead-based immunoassay for simultaneous detection of multiple fusion proteins by flow cytometry. Fusion protein-specific monoclonal antibodies (catching probes) can be covalently coupled to size- or color-coded beads. The antibody-coupled beads can be used to capture a fusion protein in the sample, and the bound fusion proteins are then detected with a cocktail of fluorochrome-conjugated, fusion protein-specific antibodies (detection probes) using flow cytometry. By utilizing appropriate size- or color-coded beads, the multiplexed immunoassay can be readily formatted into a single-tube assay for simultaneously detecting several fusion proteins within one disease category, e.g. acute myeloid leukemia (AML) or precursor-B-ALL.

W02005/015235 relates essentially to a further improvement of the method of US 6,686,165. It discloses that the sensitivity of a bead-based catching/detection assay as described in US 6,686,165 can be significantly improved by enriching a sample for fusion protein A-B relative to the native non-fused protein A and/or protein B prior to detecting the fusion protein. This is achieved by depleting a sample of one or more non-fused, native counterparts of the fusion protein.

The above mentioned fusion protein detection techniques have proven to be highly valuable for the diagnosis and monitoring of disease, in particular leukemia, lymphoma and solid tumours resulting from chromosomal aberrations that lead to fusion genes.

However, it appears that there is a need for further improvements of the method. The present invention aims to fulfil this need. For example, there are known cases wherein detection of a tumor-specific fusion gene by PCR yields a positive test result, whereas detection of the fusion gene product at the protein level yielded a negative test result. In particular, it is an object of the present invention to minimize the number of overlooked false-negative outcomes of the fusion protein assay, as well as to reduce the incidence of false-negative assay outcomes. A further object is to provide a method that allows for a reliable quantitation of one or more fusion proteins. This is especially important in inter- and intra-patient monitoring, for example to evaluate a response to anti-cancer treatment. The invention preferably aims at resolving these issues simultaneously in a simple and convenient manner. It has been found that at least some of the goals can be met by performing, in addition to the fusion protein detection analysis, one or more additional qualitative and/or quantitative control analysis, involving the use of so-called "quality control beads" and/or "quantitation beads". Further assay improvements relate to modifications of the sample preparation procedure, in particular the step of preparing a cell lysate to be assayed for the presence of one or more tumor-specific fusion proteins. As will become clear from the description below, the improvements disclosed herein are fully compatible with each other. They may be employed as such or in any combination with each other.

In one embodiment, there is provided a method for detecting a tumor-specific fusion protein, said method comprising (A) a fusion protein analysis wherein a lysate of cells suspected to contain at least one tumor-specific fusion protein is contacted with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry, the method furthermore comprising subjecting the lysate to (B) a qualitative control analysis and/or (C) a quantitative control analysis.

Step (B) comprises contacting the cell lysate with at least a bead-bound catching probe and a fluorochrome-conjugated detection probe directed against a household protein known to be present in the cells, each probe capable of recognizing distinct binding sites of said household protein, and determining binding of said probes to said household protein by flow cytometry to determine the amount of intact protein in the lysate being analyzed for the presence of a fusion protein.

Step (C) comprises contacting fluorochrome-conjugated fusion protein detection probe as used in fusion protein analysis step (A) with at least a first set of quantitation beads, the beads being provided with a known amount of binding sites for the fluorochrome-conjugated fusion protein detection probe, and measuring by flow cytometry the fluorescence signal that is associated with said known amount of binding sites. The latter can provide an indication of the amount of fusion protein detected in step (A).

Thus, in addition to the fusion protein detection analysis (A), for example employing the teaching of US 6,686,165 or WO2005/015235, a method of the invention is characterized by a qualitative control analysis (B) and/or a quantitative control analysis (C). Preferably, a method of the invention comprises analysis (A) and (B), or (A) and (C) simultaneously in a single tube.

Analysis (B) is based on the insight that upon lysis of several types of cells that are of interest to be assayed for the presence of a tumor-specific fusion protein, in particular leukocytes, a protease activity is released, which can cause an unwanted protein degradation. Especially more mature myeloid cells appear to contain granules with high levels of various proteases, which rapidly degrade the cellular proteins that are released upon cell lysis. Also the released fusion proteins appear to be degraded upon cell lysis, if (mature) myeloid cells are present in the analyzed leukocyte sample. This protease problem is particularly prominent in case of BCR-ABL fusion protein detection in cell lysates of chronic myelogenous leukemia (CML) cells. CML cells represent more mature myeloid cells and consequently can contain high levels of proteases in their granules.

If the integrity of the proteins in the cell lysate is not checked, it might well be that false-negative results are obtained because the fusion protein will not be detectable because of protein degradation. Therefore, it is important to verify the integrity of the proteins in a cell lysate to be subjected to immunobead assays, especially if the cell sample contains (more) mature myeloid cells, such as granulocytes or CML cells.

Quality control analysis (B) is advantageously performed at the time of diagnosis, when a patient sample suspected to contain a tumor-specific fusion protein is analyzed for the presence of one or more fusion proteins known to be involved in malignancies. This is typically done using a multiplex format assay, wherein different sets of probes are used to simultaneously detect different fusion proteins. The use of quality control beads in analysis (B) ensures that a possible negative assay outcome, i.e. the absence of a tumor-specific fusion protein, is interpreted correctly in the sense that conclusions can only be drawn if the cell lysate is found to be sufficient quality in terms of protein integrity.

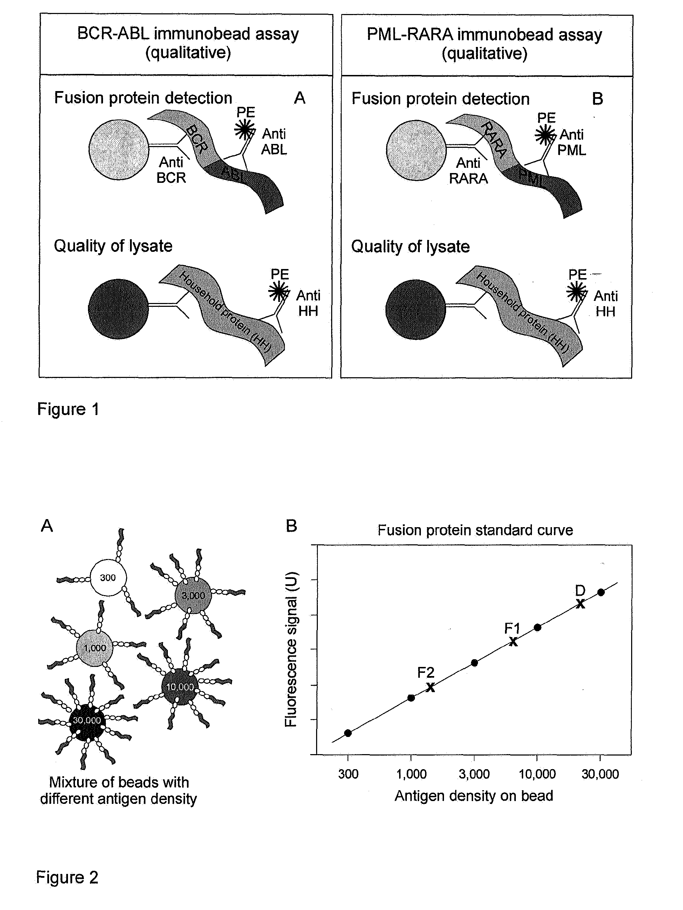

By designing a multiplex assay, e.g. using beads with different sizes and/or colours, the fusion protein and the household protein can be detected in a single tube (Figure 1). This guarantees an optimal quality control of the fusion protein detection assay. For example, if the signal of the household protein is found to be below a certain threshold level, a negative outcome of the fusion protein analysis (i.e. no detection of fusion protein) should be discarded or at least be verified by other detection methods, such as a PCR-based method for detecting the corresponding fusion gene. Alternatively, or in addition, the fusion protein analysis can be repeated using a sample of improved quality, for instance a lysate wherein protease activity is (further) inhibited and/or wherein nuclear fusion proteins are released from DNA. See herein below for details.

According to the invention, the integrity of the proteins in a cell lysate is evaluated by checking the integrity of a well-defined household protein that is stably present in the cells to be tested. For example, in leukocytes the household protein to be detected may be ABL, β2m, actin, MGUS, and the like. It is convenient to detect this household protein via a comparable immunobead assay as the fusion protein. Thus, a household protein can be detected using at least a bead-bound catching probe and a fluorochrome-conjugated detection probe directed against distinct binding sites on the household protein. The at least two household protein epitopes that are recognized by the different probes should be sufficiently separated from each other in order to have potential protease target sites between the two epitopes, so that a negative result is obtained in case of protease activity in the cell lysate. Therefore, in one embodiment the catching probe and the detection probe directed against a household protein are capable of recognizing, respectively, an N-terminal and a C-terminal binding site of said household protein, or, respectively, a C-terminal and a N-terminal binding site of said household protein. It is most preferred that the epitopes reside in a stretch of amino acids that make up the terminal 25%, preferably 15%, most preferably the terminal 10% of the household protein. Probes against a certain epitope can be produced by methods known in the art. For example, specific (monoclonal) antibody probes can be generated using a peptide immunogen comprising the epitope.

The term 'probe' refers to any moiety that comprises a portion that can specifically bind a protein target to be detected in a method of the invention, i.e. a fusion protein or a household protein. Probes may include nucleic acids, peptides, and other molecules that can specifically bind to a target. Very suitable probes are (monoclonal) antibodies or functional fragments thereof.

The term 'tumor-specific fusion protein' is meant to refer to all known and yet to be discovered proteins encoded by malignant fusion genes. Tumor-specific fusion proteins are widely known in the art. Included are those mentioned in tables 1 and 2 of US 6,686,165.

The term 'household protein' encompasses any well-defined protein that is stably present in the cell type(s) to be tested.

The term 'fluorochrome" refers to any fluorescent molecule or moiety capable of generating a detectable signal. Fluorescent dyes useful to label a probe include fluoresceins, rhodamines, benzophenoxazines, energy-transfer dyes, and cyanines.

The term 'bead' refers to any spherical particle that has the appropriate characteristics for analysis by flow cytometry. Such beads are known in the art. They may be made of any suitable material, including plastic, polystyrene, latex and other polymeric substances. To allow for multiplex assays, the different types of beads (be it fusion protein catching beads, household protein catching beads and/or different sets of quantitation beads) are preferably distinguishable from each other. For example, beads of different size and/or color are used. The term "immunobead" as used herein refers to a bead to which an antibody probe is conjugated.

A further control analysis of the invention comprises a quantitative control analysis. Quantitation of the amount of fusion protein has so far not been used in applications of a bead-based assay for detecting tumor-specific fusion proteins. Tumor-specific fusion proteins can be used for, among others, the detection of MRD, and it would be desirable to provide a method for reliable quantitation of the fusion proteins. For this purpose an elegant bead-based assay can be applied, using multiple different sets of beads (e.g. two or more sets of differently colored beads) each of them covered with different densities of the protein domain that is recognized by the detection antibody of the involved immunobead assay (e.g. anti-ABL in case of the BCR-ABL assay). Accordingly, step (C ) of a method of the invention preferably comprises the use of at least a first set of quantitation beads comprising a first known quantity of binding sites and a second set of quantitation beads comprising a second known quantity of said binding sites, to allow the plotting of a standard (or calibration) curve. As will be understood, a more reliable standard curve can be obtained using more than two sets of quantitation beads. In a preferred embodiment, a mixture of at least three sets of differently sized/colored beads is used, each set being provided with a different density of binding sites. For example, a mixture of five sets of quantitation beads is included in the assay, to obtain a standard curve ranging from 300 to 30000 binding sites (antigens) per bead. The amount of beads within a set can vary. For example, as little as 50 or 100 beads can yield a sufficient signal.

Beads can be provided with a known amount of binding sites by methods known in the art, including attaching proteinaceous binding sites suing covalent bonding, UV crosslinking, and linking through an affinity set. As a non-limiting example, a proteinaceous binding site provided with a Histag is coated onto nickel beads.

Figure 2 summarizes the mixture of the different beads and the standard curve which can be made based on the quantitation beads. This standard curve can be used for plotting the results of the fusion protein assay, thereby allowing the comparison between for example the results at diagnosis (D) and at multiple time points during follow-up (F1 and F2). In this way it becomes possible to accurately monitor the kinetics of the fusion protein levels and thereby the kinetics of the leukemic cells. Quantitative control analysis (C) is advantageously used in combination with assaying for a specific tumor-specific fusion protein during patient monitoring. However, also at the earlier step of diagnosis it may be useful to obtain information about the actual quantity of the fusion protein present prior to treatment.

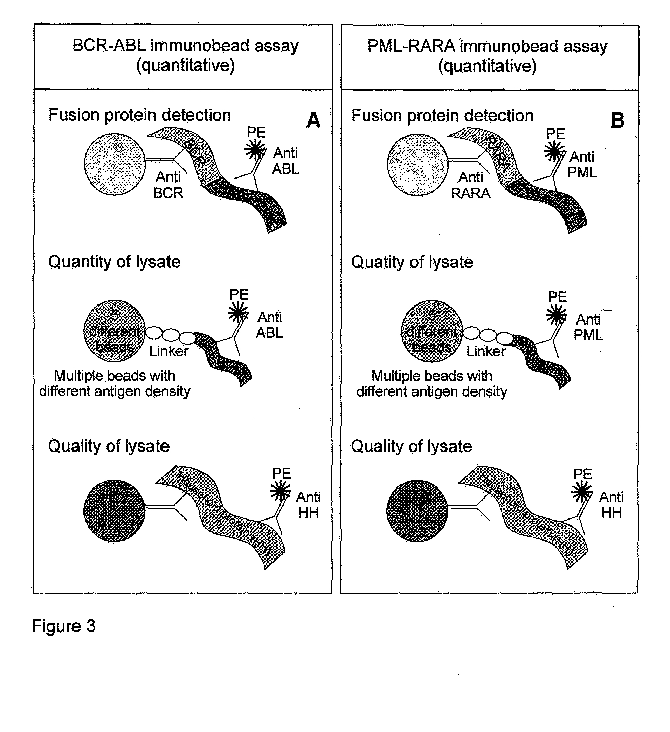

The quantitation beads can be run together with the appropriate immunobead fusion protein detection assay in a single-tube multiplex format (see Figure 3 for BCR-ABL and PML-RARA). Alternatively, it is possible to use the quantitation beads in a separate tube, for instance in a tube run parallel to multiple patient samples that are analyzed with respect to one or more tumor-specific fusion proteins.

The multiplexing possibility of a method of the invention allows for the combination of the fusion protein bead, the quality control bead (household protein bead) and the quantitation beads (see Figure 3).

The presented system is easy to establish and offers customizable and individual setup. The beads can be used for single analyte quantification or for multiplex arrays. Large quantities of distinctly colored microspheres can be manufactured at a very low cost. All components for the array can be deposited for months and can be assembled in a short time. The prepared multiplex bead array can be stored in a refrigerator and is stable for several weeks. Furthermore, arrays can be enlarged by encoding the bead subpopulations with templates of different diameters and by introducing additional fluorescence markers within the polyelectrolyte layers (e.g., Cy5). The sensitivity of the array could be further increased, e.g., by use of phycoerythrin (PE)-labeled secondary antibodies with a superior signal-to-noise ratio. Because the signal intensities increase with decreasing bead numbers at a given amount of antibodies, the performance of the system can be further optimized by adjustment for a proper particle-to-antibody ratio.

A further aspect of the invention relates to reducing the incidence of a false-negative outcome of a bead-based assay for detecting a tumor-specific fusion protein. This is achieved by means that increase the chance that a tumor-specific fusion protein which was originally present in a cell is actually being detected in the cell lysate that is subjected to a catching/detection assay. The inventors realized that several tumor-specific fusion proteins, such as PML-RARA, AML-ETO and TEL-AML1, are DNA binding molecules themselves or are involved in transcription factor protein complexes. In traditional cellular lysis methods, these nuclear proteins are not easily released from the nucleus and from the DNA bound protein complexes. Extra efforts are needed to release these fusion proteins in order to detect them with the immunobead assay. It is known in the art to release nuclear proteins by sonication. However, sonication has several drawbacks. First, it needs special laboratory facilities. Second, traditional sonicators use a probe that is directly in contact with the biological sample. This has major drawbacks in terms of reproducibility as the sonication energy depends on the depth of the sonication probe in the liquid. This hampers standardized transfer of protocols between (diagnostic) labs. Moreover, the probe system is tedious to work with, produces foam, and only one sample can be treated at a time. Also contamination between different samples is also frequently experienced. Of particular importance for the detection of tumor-specific fusion proteins, the present inventors found that a sonication step is preferably to be avoided in a method for detecting fusion proteins. For instance, the cytosolic fusion protein BCR-ABL was detected at a very low level when a lysate known to be positive for BCR-ABL was sonicated prior to analysis by a catching/detection assay. Without wishing to be bound by theory, it is thought that sonication causes (e.g. by denaturation) structural changes within the protein, thereby reducing its affinity to a specific (antibody) probe. Thus, sonication is unsuitable for use in a (multiplex) immunobead assay that aims to detect any tumor-specific fusion protein, irrespective of whether it is localized in the cytosol, the nucleus or any other cellular compartment.

It was surprisingly found that this problem can be overcome by means of an endonuclease treatment, preferably a DNAse treatment, to release or liberate nuclear fusion proteins from nucleic acids and thereby making them more accessible for recognition by and binding to a catching/detection probe set. Accordingly, the invention provides a method for detecting a fusion protein in a sample comprising cells, comprising preparing a lysate of said cells and contacting the cellular lysate with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry, wherein preparing said cellular lysate comprises the use of an endonuclease.

Several DNAses have been tested for detection of the DNA bound fusion proteins. Very good results were obtained by adding a non-specific endonuclease from *Serratia marcescens* to the lysis buffer for preparing a cellular lysate, for instance Benzonase ® from Merck KGraA, Darmstadt. Benzonase® is a genetically engineered endonuclease, see US Patent 5,173,418 and EP Patent No. 0229866. A person skilled in the art can determine the concentration of endonuclease required to release a nuclear fusion protein. Preferably, Benzonase is used at a concentration of at least 10 mU/ml, preferably at least 20 mU/ml, such as 25 mU/ml or higher.

The Benzonase protocol was tested on several other fusion protein assays (e.g. ABL-BCR). These experiments demonstrated that, in contrast to the sonication procedure, the endonuclease treatment has no or only minor negative effects on the outcome of the immunobead assay for cytosolic tumor-specific fusion proteins (no or only a very minor reduction of signal). Therefore, endonucleases like Benzonase are advantageously included in the routine protocol for fusion protein detection, irrespective of the type or subcellular localization of the fusion protein. This is particularly important for the multiplex assays wherein various different tumor-specific fusion proteins are detected simultaneously in a single tube.

The use of endonucleases is known in the art primarily with respect to subcellular fractionation, such as in methods for obtaining partial proteomes from the complete proteome of a cell preparation. US 7,262,283 discloses the use of Benzonase ® for the extraction of "insoluble" nuclear proteins (e.g., histones) in a method for the sequential production of a partial proteome enriched with cytosolic proteins, partial proteome enriched with membrane/organelle proteins, partial proteome enriched with proteins from the cell nucleus interior, partial proteome enriched with proteins of the cytoskeleton and of the nuclear matrix. The study of Martine et al. (BMC Cancer. 2004; 4: 4) relates to fusion protein RUNX1-CBFA2T1 associated with t(8;21)-positive acute myeloid leukaemia. To obtain nuclear lysates for immunoblotting analysis of RUNX1-CBFA2T1, cells were washed in a lysis buffer comprising Benzonase. However, the prior art does not teach or suggest the use of an endonuclease in the preparation of a total cell lysate to be analyzed for a variety of tumor-specific fusion proteins using a bead-based catching/detection assay as disclosed herein.

A further solution to the problem of masked fusion protein detection as provided herein relates to the inhibition of protein degradation. As mentioned herein above, protease activity severely affects the integrity of the fusion proteins in a bead-based (immuno)detection assay. This problem is particularly prominent when (more) mature myeloid cells are present in the patient sample. Particularly in patient samples with high frequencies of granulocytes or CML cells, false-negative results can be obtained due to protein degradation. For example, BCR-ABL is readily detectable in pre B-ALL cells and in tumor cell lines such as K562. However, in CML patients the detection of BCR-ABL is severely hampered, presumably due to granulocyte proteases, such as cathepsin, protease 3 and elastase.

A series of protocol optimization experiments performed by the present inventors revealed that degradation of tumor-specific proteins was only effectively inhibited when protease inhibitors are added to the intact cells before cell lysis (i.e. in a preincubation step employing a non-lytic buffer) as well as during cell lysis (i.e. in the lysis buffer). This combined approach results in a surprisingly strong reduction of protease activity and thereby contributes to the prevention of false-negative results.

Accordingly, the invention provides a method for detecting a fusion protein in a sample comprising cells, comprising preparing a lysate of said cells and contacting the cellular lysate with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry, wherein preparing the cellular lysate comprises a treatment of intact (viable) cells with at least one cell permeable protease inhibitor, followed by lysis of said pretreated cells in a lysis buffer comprising one or more protease inhibitors. In one embodiment, the cell sample comprises white blood cells, granulocytes or a mixture thereof. Preferably, the intact cells are incubated with at least one irreversible serine protease inhibitor. Very suitable serine protease inhibitors are phenylmethyl sulfonyl fluoride (PMSF) and 4-(2-Aminoethyl) benzenesulfonyl fluoride hydrochloride (AEBSF HCl). AEBSF is a water soluble, irreversible serine protease inhibitor with a molecular weight of 239.5 Da. It inhibits proteases like chymotrypsin, kallikrein, plasmin, thrombin, and trypsin. The specificity is similar to the inhibitor PMSF, nevertheless AEBSF is more stable at low pH values. PMSF is a serine protease inhibitor but it does not inhibit all serine proteases. PMSF is rapidly degraded in water and stock solutions are usually made up in anhydrous ethanol, isopropanol, corn oil, or DMSO.

The combined use of PMSF and AEBSF was found to result in a significant reduction of protease activity, particularly when combined with extra protease inhibitors in the lysis buffer (see Example 1). Therefore, preferably a combination of PMSF and AEBSF is used for pretreating intact cells. PMSF may be used at various concentrations, for instance 0.5 to 5 mM final, preferably 0.5-2 mM, such as around 1 mM final. AEBSF is typically used at a higher concentration, for example 5-50 mM, preferably 10-30 mM, such as around 20 mM.

The addition of extra protease inhibitors in the lysis buffer serves to further reduce the protease activity in the lysate. In a preferred embodiment, the lysis buffer is supplemented with a standard cocktail of several types of (broad spectrum) protease inhibitors. Since cells have different types of proteases, mixtures of different protease inhibitors are usually required to maintain and preserve cellular protein composition following cell lysis. A typical cocktail contains a mixture of water-soluble protease inhibitors with broad specificity for the inhibition of serine, cysteine, aspartic, and metalloproteases. These are well known in the art, and obtainable from various commercial sources. For example, the Protease Inhibitor Cocktail marketed by BD Biosciences Pharmingen may be used. This 50 x concentrated cocktail contains benzamidine HCl, phenanthroline, aprotinin, leupeptin, pepstatin and PMSF. Another commercially available protease mixture for supplementing the lysis buffer contains AEBSF, E-64, bestatin, leupeptin, aprotinin, and sodium EDTA. As will be understood, the use of other combinations of protease inhibitors in a method of the invention is also encompassed.

A further aspect of the invention relates to kits for use in a method for detecting a fusion protein as disclosed. As will be understood, a kit of the invention can contain various components depending on the specific application (e.g. diagnosis, follow-up during treatment) desired.

In one embodiment, the kit comprises,
- a probe set (A) comprising at least a first bead-bound fusion protein catching probe (A1) and a second fluorochrome-conjugated fusion protein detection probe (A2), each probe capable of recognizing a binding site positioned at opposite sides of the fusion region of said fusion protein,
- a probe set (B) comprising a bead-bound household protein catching probe (B1) and a fluorochrome-conjugated household protein detection probe (B2), each probe capable of recognizing binding sites positioned at distinct sites on a household protein expressed in said cell; and/or
- at least a first set of quantitation beads (C1), the beads being provided with a first known amount of binding sites for fusion protein detection probe (A2).

Preferred probes are antibodies or functional fragments thereof. However, other types of specific binding molecules that can be conjugated to a bead and/or fluorochrome are also encompassed.

The kit may furthermore comprise a second set of quantitation beads (C2), the beads being provided with a second known amount of binding sites for probe (A2). Together with the first set of quantitation beads, this allows for the plotting of a standard curve. Preferably, the kit comprises at least three, more preferably at least four sets of quantitation beads, each set being provided with a different amount of binding sites for the fusion protein detection probe. It is of course to be understood that among the multiple sets of quantitation beads there is a significant difference in the amount of binding sites, e.g. at least a two- or three-fold difference, between different sets of beads. Furthermore, it is preferred that the quantitation beads cover a range of binding sites that is or can be expected to cover the number of fusion protein to be detected in a sample. For example, a kit of the invention comprises five sets of beads, spanning a range of about 300 to about 30.000 binding sites per bead. For single tube assaying of the multiple sets of quantitation beads, it is preferred that the beads belonging to the different sets are distinguishable from each other, for instance on the basis of size and/or color. In that case, the kit may contain a mixture of two or more sets of quantitation beads. Most preferred are kits comprising differently colored sets of quantitation beads, like Luminex ™ beads. Other useful kit components include buffers, lysing and/or washing reagents.

To allow for the detection of multiple tumor-specific fusion proteins, a kit preferably comprises at least two probe sets (A), each probe set being capable of specifically binding to and detecting a different tumor-specific fusion protein. For example, a kit comprises a bead-bound anti-BCR antibody and a fluorochrome-conjugated anti-ABL antibody for detecting BCR-ABL, as well as a bead-bound anti-RARA antibody and a fluorochrome-conjugated anti-PML antibody for detecting PML-RARA. Again, it is preferred that the different beads, i.e. the anti-BCR bead and the anti-PML bead, are distinguishable by flow cytometry. Of course, any combination of tumor-specific probe sets can be present in a kit of the invention. In one aspect, it comprises a probe set (A) directed against at least one, preferably at least two, tumor-specific fusion protein(s) selected from the group consisting of MLL-AF4, MLL-AF6, MLL-AF9, MLL-ENL, MLL-AF10, MLL-ELL, PML-RARA, PLZF-RARA, NPM-RARA, NUMA-RARA, NPM-ALK, TPM3-ALK, TFG-ALK, ATIC-ALK, EWS-FLI1, EWS-ERG, EWS-ETV1, AML1-ETO, PML-RARA, CBFB-MYH11, E2A-PBX1, BCR-ABL and TEL-AML1.

Probe set (B) in a kit of the invention is directed against a household protein. For instance, it comprises a bead-bound catching antibody and a fluorochrome-conjugated antibody directed at distinct sites of a household protein selected from the group consisting of ABL, β2M and GUS,...[Q: others to be mentioned here?]. To increase the reliability of the qualitative control analysis (B), it is preferred that the binding sites (epitopes) for the probe set (B) are sufficiently separated from each other, thereby covering as many protease sites as possible. For instance, they each reside in a stretch of amino acids that make up the terminal 25%, preferably 15%, most preferably the C- resp. N-terminal 10% of the household protein.

A further aspect of the invention relates to a kit for the flow cytometric detection of at least one tumor-specific fusion protein in a cell, the kit comprising a probe set (A) comprising at least a first bead-bound fusion protein catching probe (A1) and a second fluorochrome-conjugated fusion protein detection probe (A2), each probe capable of recognizing a binding site positioned at opposite sides of the fusion region of said fusion protein, wherein the kit furthermore comprises an endonuclease. As is described herein above, the endonuclease is advantageously used to enhance the detection of DNA-binding tumor-specific fusion proteins without the need for sonication. Preferably, the kit comprises a nonspecific endonuclease from *Serratia marcessens*, more preferably Benzonase ®.

Still a further aspect relates to a kit comprising a first container comprising at least one cell permeable irreversible serine protease inhibitor, preferably phenylmethyl sulfonyl fluoride (PMSF) or 4-(2-Aminoethyl) benzenesulfonyl fluoride hydrochloride (AEBSF HCl), more preferably a combination of PMSF and AEBSF, and a second container comprising a protease inhibitor cocktail. The inhibitor cocktail may be present as part of a cell lysis buffer contained in the kit.

The sensitivity of a fusion protein detection assay can be improved by subjecting a cell lysate to a so-called "pre-clear step" disclosed in W02005/015235. Accordingly, a kit of the present invention may additionally comprise at least one bead-bound binding molecule specifically reactive with a native protein, wherein a fragment of said native protein is part of the tumor-specific protein to be detected and wherein said binding molecule is reactive with the native protein but not with the fusion protein. In one embodiment, these so-called "preclear beads" are reactive with a fragment of BCR or ABL that is not present in the BCR-ABL fusion protein.

A kit as disclosed herein is of use in many clinical diagnostic application, in particular for the diagnosis, classification and/or monitoring of a disease. Provided is for instance a "Diagnosis kit" specifically designed for performing an immunobead assay on a patient sample suspected to contain a tumor-specific fusion protein but wherein the identity of said fusion protein has not yet been revealed. A Diagnosis kit typically comprises multiple probe sets A for simultaneous detection of multiple tumor-specific fusion proteins and qualitative control beads to evaluate the integrity of a cell lysate. The kit preferably also comprises PMSF, AEBSF and an endonuclease, like Benzonase^{™}, for the preparation of a cell lysate wherein both the quality and quantity of the fusion proteins, if present, are maximized.

A further embodiment relates to a "Monitoring kit" specifically designed for disease monitoring, e.g. for the follow-up of an MRD patient known to have a certain fusion protein and receiving therapy. A Monitoring kit typically comprises a probe set for detecting a defined fusion protein, like BCR-ABL, , quantitative control beads capable of binding the fusion protein detection probe (e.g. beads coated at a known density with a fragment of BCR that is recognized by fluorochrome-conjugated anti-BCR antibody). It preferably also comprises the appropriate preclear beads capable of depleting the native non-fused protein (e.g. BCR or ABL) to remove competing native protein from the lysate, thereby enhancing the detection sensitivity. Similar to the Diagnosis kit, it may furthermore comprise PMSF, AEBSF and an endonuclease, like Benzonase^{™}.

### Legends to the Figures

Figure 1: schematic representation of fusion protein detection and qualitative control analysis. Upper parts show detection of a tumor-specific fusion protein (panel A: BCR-ABL; panel B: RARA-PML) using a bead-bound catching probe directed against the BCR- resp. RARA- fragment of the fusion protein, and a fluorochrome (in this case PE)-conjugated detection probe capable of recognizing the ABL resp. PML-fragment of the fusion protein. In parallel, preferably in the same tube, the integrity of the lysate is evaluated using qualitative control beads capable of recognizing and catching a household protein (HH) and a fluorochrome (PE)-conjugated HH detection probe (panels 3 and 4). The use of distinguishable beads (in this case indicated by a different gray shading of the beads) used in panels 1-4 allows for the simultaneous detection of different fusion proteins and a household protein in a single tube.
Figure 2: panel A shows a schematic drawing of five sets of quantitation beads, each set of beads being provided with a different amount of binding sites (antigens) for the tumor-specific detection probe. Panel B shows an exemplary standard curve that can be obtained using the quantitation beads. This standard curve can be used for plotting the results of the fusion protein assay, thereby allowing the comparison between for example the results at diagnosis (D) and at multiple time points during follow-up (F1 and F2). In this way it becomes possible to accurately monitor the kinetics of the fusion protein levels and thereby the kinetics of the leukemic cells.
Figure 3 : The quantitation beads can be run together with the involved immunobead assay (in this case BCR-ABL (panel A) and PML-RARA (panel B) detection) in a multiplex format. Alternatively, it is possible to use the quantitation beads in a separate tube in parallel to multiple patient samples that are analyzed with the involved immunobead assay. The quality of the lysate can be checked by detecting the amount of intact household protein. As in Figures 1 and 2, the different "grey colors" of the beads indicate that the different beads can be recognised during flow cytometry, while the fluorochrome of the detection antibodies can be the same.

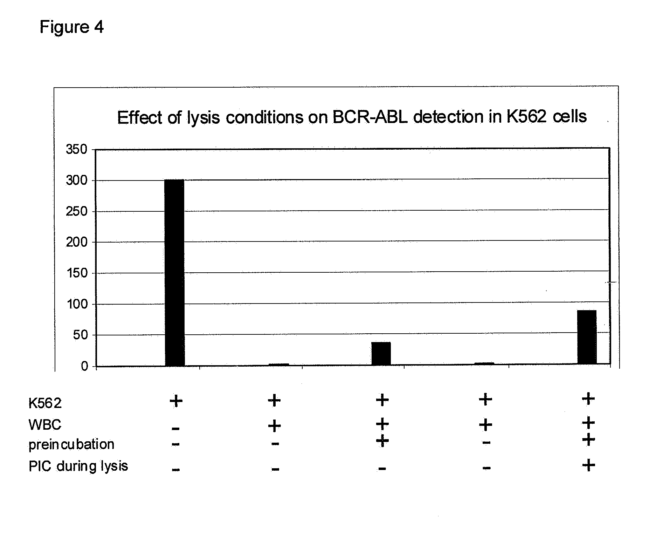

Figure 4: Effect of pretreating intact cells with protease inhibitors prior to cell lysis on the detection of BCR-ABL in K562 cells. For details, see Example 1. The Y-axis shows the BCR-ABL signal to noise ratio.

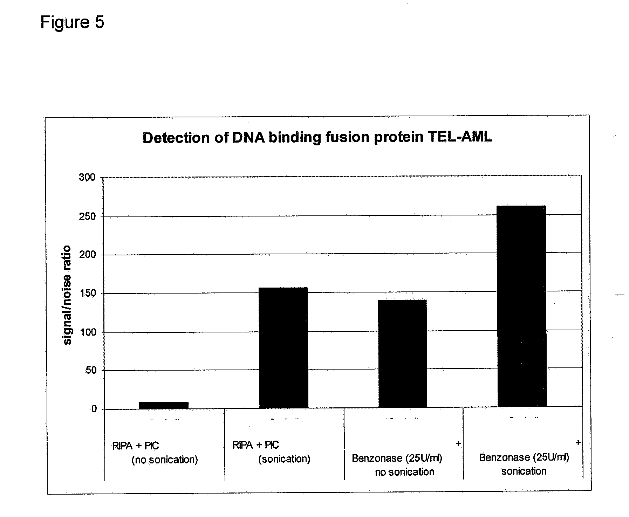

Figure 5: Effect of endonuclease treatment on the detection of a DNA-binding tumor-specific fusion protein TEL-AML. For details, see Example 2. The Y-axis shows the TEL-AML signal to noise ratio.

### EXAMPLES

### Example 1: Effect of Protease inhibitors (pre-treatment and/or during lysis) on BCR-ABL fusion protein detection.

**Cells:** K562 is a Ph⁺ cell line obtained from a CML patient during blast crisis. The cell line expresses the p210 isoform of the BCR-ABL fusion protein. 697, is a protease negative, Ph- leukemic cell line that contains the (t(1;19)) translocation. Total white blood cells (WBC) were obtained from whole blood of a healthy donor after NH₄Cl lysis of the erythrocytes. Blood was collected after informed consent of the healthy donor.

**Test samples:** To mimic a leukemic sample, cells of the Ph⁺ positive cell line K562 were mixed with WBC of a healthy donor (containing a large amount of proteases). As a control K562 cells were mixed in a 1:4 ratio with cells of the Ph⁻ cell line 697, which contains only little protease activity. Prior to lysis, the still intact cells were incubated on in ice in the presence or absence of cell permeable protease inhibitors. Hereafter the cells were resuspended in a lysis buffer with or without protease inhibitors (PIC). After incubation on ice the cell lysate was spun down and the supernatant was used for a bead assay which detects the presence of the intact BCR-ABL fusion protein

**Pretreatment of the cells with cell permeable protease inhibitors:** To inhibit protease activity during cell lysis, intact cells were pretreated with cell permeable protease inhibitors. Pelleted cells were resuspended (maximum 10⁷ cells/ml) in 1 ml pretreatment buffer supplemented with 20 mM AEBSF (4=(2-Aminoethyl)benzenesulfonyl fluoride hydrochloride, Sigma Aldrich, Zwijndrecht, The Netherlands) and 1or 5 mM PMSF (Phenylmethanesulfonyl fluoride, Sigma Aldrich) in PBS) and incubated for 10 minutes on ice. Hereafter the cells were spun down for 5 minutes at 520 G at 4°C.

**Generation of a cell lysate:** The pelleted cells were resuspended (25 million cells/ml, unless stated otherwise) in RIPA buffer (50 mM Tris HCL pH 7.4, 150 mM NaCl, 1%NP40, 0.5% Sodiumdeoxycholate, 1 mM EDTA, 0.1% SDS and 0.01% NaN₃) in the presence or absence of a protease inhibitor cocktail (PIC, Sigma Aldrich, product nr. P2714). After 30 minutes on ice, the lysate was spun down to remove cell debris and the supernatant of the lysate was used in a flow cytometric bead assay.

**Bead Assay:** To a filterplate (Millipore), 50 µl of lysate, 25 µl beads provided with anti-BCR antibody (catching probe) beads and 50 µl PE-conjugated anti-ABL antibody (detection probe) in PBS/1% BSA were added (6000 beads in total) and incubated for two hours at room temperature while shaking. The filterplate was washed three times with PBS/1% BSA and drained. Subsequently, 1000 beads were acquired on a FACS Canto II flow cytometer (BD Biosciences).

For all different incubation conditions, the BCR-ABL bead assay was performed according to the standard protocol. For quantification the ratio was calculated between the mean fluorescence signal detected in the lysates of the K562 cells treated with the various incubation conditions and the mean fluorescence signal obtained when the assay (including pre-treatment, lysis and bead assay) is performed on a cell lysate of pure WBC of a healthy individual (background value).

**Results** Figure 4 shows that BCR-ABL is readily detectable in a cell lysate of protease poor K562 cells, even if no protease inhibitors are used. In contrast, lysis of K562 cells in the presence of granulocyte-rich WBC's results in a nearly complete loss of the detected signal. Preincubation of this cell mixture with AEBSF and PMSF partially restores the signal, whereas the mere addition of a protease inhibitor cocktail to the lysis buffer has no effect. Surprisingly, the combination of intact cell pretreatment and supplementing lysis buffer with the cocktail has a synergistic effect on the detection signal. Whereas the BCR-ABL signal is not fully restored to the level obtained in the absence of protease-rich cells, it is sufficient for a reliable assay outcome.

### Example 2: Endonuclease treatment can replace sonication.

The tumor-specific fusion protein TEL-AML is a DNA binding protein and can only be detected by a TEL-AML specific bead assays after sonication of the lysate. Since this procedure is not applicable in all standard diagnostic laboratories, the use of DNAses to digest DNA and make the TEL-AML (and other) fusion proteins accessible for detection was investigated.

**Cells:** REH t(12;12) is a cell line in which the oncogenic fusion protein TEL-AML is expressed.

**Generation of a cell lysate:** The pelleted cells (25 million cells/ml RIPA) were resuspended in RIPA buffer (50 mM Tris HCL pH 7.4, 150 mM NaCl, 1%NP40, 0.5% Sodiumdeoxycholate, 1 mM EDTA, 0.1% SDS and 0.01% NaN₃) to which a protease inhibitor cocktail (PIC, Sigma Aldrich) is added in the presence or absence of the non-specific endonuclease Benzonase ™ (Novagen, Merck KGaA Darmstadt, Germany). Part of the lysates were sonicated and after 15 minutes incubation on ice, the lysate was spun down to remove cell debris and the supernatant was used in a cytometric bead assay.

### Test samples:

REH cells were spun down and the pelleted cells were resuspended in RIPA lysis buffer with added protease inhibitors (PIC). Here after
**Sample 1**: 15 minutes incubation on ice
**Sample 2**: cells were sonicated, followed by incubation on ice for 15 minutes
**Sample 3:** 25 U/ml of benzonase was added to the lysis buffer and cells were incubated for 15 minutes on ice.
**Sample 4**: 25 U/ml of benzonase was added to the lysis buffer, cells were sonicated and incubated for 15 minutes on ice.

After incubation, the various lysates were spun down and the supernatants were subjected to a TEL-AML specific catching/detection bead assay performed under standard conditions (see Example 1).

For quantification of the TEL-AML detection signal, the ratio was calculated between the mean fluorescence signal detected for the various cell lysates and the background value. The background value is the mean fluorescence signal obtained when the assay (including pre-treatment, lysis and bead assay) is performed on a lysate of a cell line known to be negative for the TEL-AML fusion protein.

**Results:** Figure 5 demonstrates that only a very low TEL-AML signal is detected in a lysate prepared using a lysis buffer with protease inhibitors only. Sonication of the lysate dramatically enhances the TEL-AML signal. Supplementing the lysis buffer with an endonuclease has essentially the same effect. The endonuclease treatment combined with sonication even further enhances the signal. However, the signal-to-noise-ratio obtained with endonuclease alone is more than sufficient for a reliable assay outcome. Sonication is preferably to be avoided in case also one or more cytosolic fusion proteins, such as BCR-ABL, are to be detected in the lysate.

## Claims

1. A method for detecting a tumor-specific fusion protein, said method comprising a fusion protein analysis (A) wherein a lysate of cells suspected to contain at least one tumor-specific fusion protein is contacted with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry,
the method furthermore comprising subjecting the lysate to a qualitative control analysis (B) and/or a quantitative control analysis (C),
wherein said qualitative internal control analysis (B) comprises contacting lysate with at least a bead-bound catching probe and a fluorochrome-conjugated detection probe directed against a household protein known to be present in the cells, each probe capable of recognizing distinct binding sites of said household protein, and determining binding of said probes to said household protein by flow cytometry to determine the amount of intact protein in the lysate analyzed for the presence of the fusion protein; and
wherein said quantitative control analysis (C) comprises contacting the fluorochrome-conjugated fusion protein detection probe as used in analysis (A) with at least a first set of quantitation beads being provided with a first known amount of binding sites for the fluorochrome-conjugated fusion protein detection probe, and measuring by flow cytometry the fluorescence signal that is associated with said known amount of binding sites to determine the amount of fusion protein detected in step (A).

2. The method according to claim 1, wherein fusion protein analysis (A) and control analysis (B) and/or (C) are performed simultaneously in a single tube using beads that are distinguishable during flow cytometric detection.
Quantitation beads different colors distinguishable by FACS. Distinctly colored beads, Luminex TM

3. The method according to claim 1 or 2, wherein the bead-bound catching probe and a fluorochrome-conjugated detection probe directed against a household protein used in qualitative control analysis (B) are capable of recognizing, respectively, an N-terminal and a C-terminal binding site of said household protein, or, respectively, a C-terminal and a N-terminal binding site of said household protein.

4. Method according to any one of the preceding claims, wherein said household protein is selected from ABL, β2M, MGUS, etc.

5. Method according to any one of the preceding claims, wherein quantitative control analysis (C) comprises the use of at least a first set of quantitation beads comprising a first known amount of binding sites and a second set of quantitation beads comprising a second known amount of said binding sites, to allow the plotting of a standard curve. *In text: At least three, at least four, at least five*.

6. A method for detecting a fusion protein in a sample comprising cells, comprising preparing a lysate of said cells and contacting the cellular lysate with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry,
wherein preparing said cellular lysate comprises contacting cells with a lysis buffer supplemented with an endonuclease.

7. Method according to claim 6, wherein said endonuclease is a nonspecific endonuclease from *Serratia marcessens*.

8. Method according to claim 6 or 7, comprising the detection of a cytosolic and a nuclear fusion protein.

9. A method for detecting a fusion protein in a sample comprising cells, comprising preparing a lysate of said cells and contacting the cellular lysate with at least a bead-bound catching probe and a fluorochrome-conjugated detection probe directed against the at least one fusion protein, each probe capable of recognizing a binding site positioned at opposite sides of the fusion region of said fusion protein, and determining binding of said probes to said fusion protein by flow cytometry,
wherein preparing the cellular lysate comprises a treatment of intact cells with at least one cell permeable protease inhibitor, followed by lysis of said pretreated cells in a lysis buffer comprising one or more protease inhibitors.

10. Method according to claim 9, wherein the intact cells are incubated with at least one irreversible serine protease inhibitor.

11. Method according to claim 10, wherein intact cells incubated with at least one of phenylmethyl sulfonyl fluoride (PMSF) and 4-(2-Aminoethyl) benzenesulfonyl fluoride hydrochloride (AEBSF HCl), preferably a combination thereof.

12. Method according to any one of the preceding claims, wherein fusion protein analysis (A) is preceded by depleting the lysate of at least one native protein that can compete with a tumor-specific fusion protein for binding to a probe used in protein analysis (A), wherein said depleting comprises contacting the lysate with at least one bead-bound binding molecule that is reactive with a native protein, wherein a fragment of said native protein is part of a tumor-specific protein to be detected and wherein said binding molecule is reactive with the native protein but not with the fusion protein. *[NB: this reads onto preclear procedure]*

13. Method according to any one of the preceding claims, comprising detecting at least two different tumor-specific fusion proteins simultaneously using a specific set of bead-bound catching probes and fluorochrome-conjugated detection probes for each of the fusion proteins.

14. Method according to any one of the preceding claims, comprising detection of one or more, preferably at least two, tumor-specific proteins selected from the group consisting of MLL-AF4, MLL-AF6, MLL-AF9, MLL-ENL, MLL-AF10, MLL-ELL, PML-RARA, PLZF-RARA, NPM-RARA, NUMA-RARA, NPM-ALK, TPM3-ALK, TFG-ALK, ATIC-ALK, EWS-FLI1, EWS-ERG, EWS-ETV1, AML1-ETO, PML-RARA, CBFB-MYH11, E2A-PBX1, BCR-ABL and TEL-AML1.

15. A kit for the flow cytometric detection of a tumor-specific fusion protein in a cell, the kit comprising
- a probe set (A) comprising at least a first bead-bound fusion protein catching probe (A1) and a second fluorochrome-conjugated fusion protein detection probe (A2), each probe capable of recognizing a binding site positioned at opposite sides of the fusion region of said fusion protein,
- a probe set (B) comprising a bead-bound household protein catching probe (B1) and a fluorochrome-conjugated household protein detection probe (B2), each probe capable of recognizing binding sites positioned at distinct sites on a household protein expressed in said cell; and/or
- at least a first set of quantitation beads (C1), the beads being provided with a first known amount of binding sites for fusion protein detection probe (A2).

16. Kit according to claim 15, furthermore comprising a second set of quantitation beads (C2), the beads being provided with a second known amount of binding sites for probe (A2).

17. Kit according to claim 15 or 16, comprising at least two probe sets (A), each probe set being capable of specifically binding to and detecting a different tumor-specific fusion protein.

18. Kit according to any one of claims 15-17, comprising a probe set (A) directed against at least one, preferably at least two, tumor-specific fusion protein(s) selected from the group consisting of MLL-AF4, MLL-AF6, MLL-AF9, MLL-ENL, MLL-AF10, MLL-ELL, PML-RARA, PLZF-RARA, NPM-RARE, NUMA-RARA, NPM-ALK, TPM3-ALK, TFG-ALK, ATIC-ALK, EWS-FLI1, EWS-ERG, EWS-ETV1, AML1-ETO, PML-RARA, CBFB-MYH11, E2A-PBX1, BCR-ABL and TEL-AML1.
*Probes are antibodies or functional fragments thereof in text*

19. Kit according to any one of claims 15-18, wherein the beads of probe set (A), beads of probe set (B) and/or quantitation beads (C) have different bead characteristics, preferably a different size and/or color.

20. Kit according to any one of claims 15-19, wherein probe set (B) is directed against a household protein selected from the group consisting of ABL, β2M, GUS,...**[Q: others to be mentioned here?]**

21. Kit according to any one of claims 15-20, furthermore comprising an endonuclease, preferably a nonspecific endonuclease from *Serratia marcessens*, more preferably Benzonase ®.

22. Kit according to any one of claims 15-21, furthermore comprising at least one cell permeable irreversible serine protease inhibitor, preferably phenylmethyl sulfonyl fluoride (PMSF) or 4-(2-Aminoethyl) benzenesulfonyl fluoride hydrochloride (AEBSF HCl), more preferably a combination thereof.

23. Kit according to any one of claims 15-22, furthermore comprising at least one bead-bound binding molecule specifically reactive with a native protein, wherein a fragment of said native protein is part of the tumor-specific protein to be detected and wherein said binding molecule is reactive with the native protein but not with the fusion protein. *[NB: this reads onto preclear beads]*.

24. Use of a kit according to any one of claims 15-23 in the diagnosis, classification and/or monitoring of a disease.
